(19) Europäisches Patentamt
European Patent Office
Office européen des brevets

(11) Publication number: **0 389 813 B1**

# (12) EUROPEAN PATENT SPECIFICATION

(45) Date of publication of patent specification: **17.08.94**

(51) Int. Cl.5: **C07D 491/107**, //C09K9/02, G11B7/24

(21) Application number: **90103825.7**

(22) Date of filing: **27.02.90**

## (54) A method for preparing a photochromic material.

(30) Priority: **30.03.89 JP 80360/89**

(43) Date of publication of application:
**03.10.90 Bulletin 90/40**

(45) Publication of the grant of the patent:
**17.08.94 Bulletin 94/33**

(84) Designated Contracting States:
**DE FR GB**

(56) References cited:
**FR-A- 2 114 747**
**FR-A- 2 445 334**

**Chemical Abstracts, vol. 83, no. 1, July 7, 1975, Columbus, Ohio, USA SAMOILOVA, N.P. et al. "1,1,3-Trimethyl-5,6'-dinitrospiro ((2'H)-1' - benzopyran 2,2- indoline)" page 818, column 1, abstract-no 9 829y & Otkrytiya Izobret., Prom. Obraztsy. Tovarnye Znaki 1975, 52(1), 52 U.S.S.R. 455,955**

**A. Hinnen, Ch. Audic, R. Gautron, Bull. Soc. Chim. Fr. No. 5 (1968), 2066 - 2074**

(73) Proprietor: **MATSUSHITA ELECTRIC INDUSTRIAL CO., LTD.**
**1006, Oaza Kadoma**
**Kadoma-shi, Osaka-fu, 571 (JP)**

(72) Inventor: **Hibino, Junichi**
**Azuma Apt. 201,**
**46-2 Kuzuhanaka-machi**
**Hirakata-shi, Osaka (JP)**
Inventor: **Ando, Eiji**
**4-15-20 Fujigao**
**Katano-shi, Osaka (JP)**

(74) Representative: **Schwabe - Sandmair - Marx**
**Stuntzstrasse 16**
**D-81677 München (DE)**

EP 0 389 813 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a method for preparing a photochromic material for use in optical storage media and other areas of application.

Substances which display reversible color changes upon exposure to light are collectively known as photochromic materials. Spiropyran compounds constitute one of the most intensively studied types of photochromic material.

Many spiropyran compounds have already been reported in literatures. For example, the colorless spiropyran compound A of the following formula is transformed into the red compound merocyanine B by irradiation with ultraviolet rays with a wavelength of approximately 340 nm. The compound B reverts to the form A if irradiated with visible light with a wavelength of approximately 580 nm.

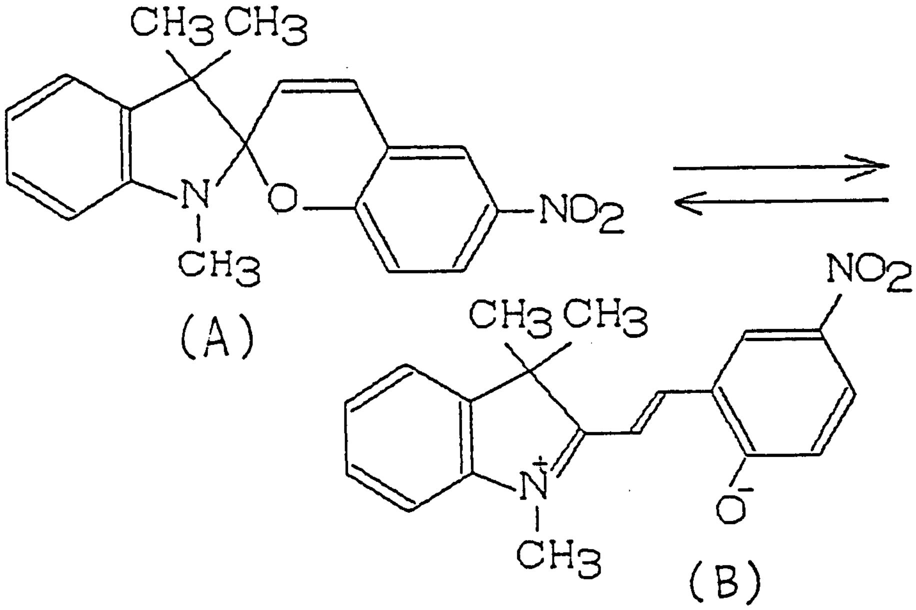

(A) (B)

Optical storage media can be prepared by utilizing these photochromic materials which change their structures by irradiation. In order to miniaturize devices containing optical storage media, the use of semiconductor lasers is generally desirable. The semiconductor lasers employed in optical storage devices ordinarily emit light in a wavelength region in the neighborhood of 700 nm, and therefore the colored form of photochromic materials used for such purposes should desirably possess absorption sensitivity in this wavelength region.

Furthermore, the formation of uniform ultrathin films of photochromic material on an appropriate substrate is desirable in order to obtain optical storage media capable of accurately recording information with high sensitivity and stably storing this information for long periods of time. The Langmuir-Blodgett method (hereinafter referred to as the LB method) is the most suitable method for obtaining such ultrathin films under gentle conditions.

With a view to forming thin films by the LB method, the spiropyran compounds used should desirably possess a balanced combination of both hydrophilic and hydrophobic moieties. However, the spiropyran skeleton alone is of low hydrophobicity, and therefore does not permit the satisfactory formation of thin films by the LB method. Many spiropyran compounds incorporating various types of substituents including hydrophilic and hydrophobic groups have been proposed with a view to solving this problem. For example, M. Morin et al. (Can. J. Chem., 58, 2038 (1980)) disclosed spiropyran compounds with long alkyl chains introduced at the N position of the indoline ring of the spiropyran skeleton. Also, spiropyran compounds with substituent nitro groups on the indoline ring are also known. These spiropyran compounds with nitro groups on the indoline ring have colored forms with absorptions in the long wavelength region, are also of high stability, and possess excellent properties for recording information.

However, the introduction of a long-chain alkyl group at the N position of an indoline ring with nitro groups is extremely difficult because of the electron attractive property of the nitro groups. Consequently, there has hitherto been no example of a spiropyran compound having a long-chain alkyl group at the 1' position of the spiropyran skeleton and a nitro group on the indoline ring (in particular, at the 5' position of

the spiropyran skeleton).

In the document "A. Hinnen et al., Bulletin de la Société Chimique de France 1968, pages 2066 et seq.", there is disclosed a process for the preparation of indolospiropyranes by condensation of 2-alcoylindenes with salicylaldehyde in an alcohol as the solvent and optionally in the presence of a small amount of piperidine. However, reaction products with long-chain alkyl residues at the nitrogen atom (R = $C_{>5}$ in the general formula II) could not be obtained by the process of said document.

Compounds of the general formula II of the present specification, wherein R is $CH_3$ and which were obtained by treating similar compounds, wherein no nitro group is present in the indol part of the molecule, with acidic aqueous $NaNO_2$ in air, were reported in C. A. 83 (1975), page 818, ref. no. 9829y. No reference is made to compounds with long-chain alkyl residues at the ring-nitrogen atom.

Similar compounds with substitution at the ring-nitrogen atom by lower alkyl residues are also disclosed in FR-A 2,445,334. Said document, however, is not concerned with compounds of the present invention having alkyl residues as substituents at the ring-nitrogen atom wherein the number of carbon atoms is ≧ 6.

The method for preparing a photochromic material of this invention, which overcomes the above-discussed and numerous other disadvantages and deficiencies of the prior art, comprises the step of subjecting a spiropyran compound of formula I to direct nitration to form a nitrated spiropyran compound of formula II which functions as a photochromic material:

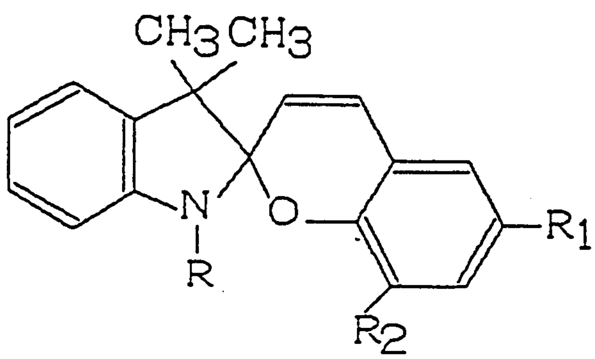

(I)

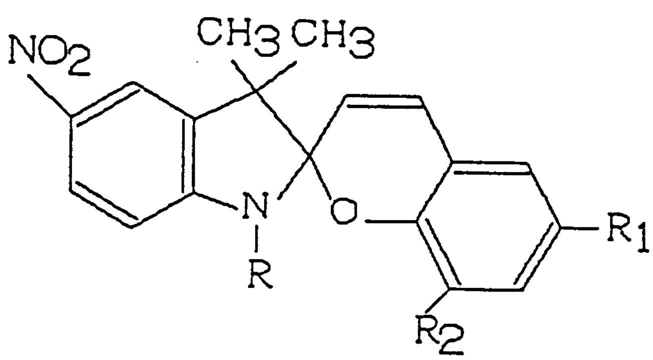

(II)

wherein R is $C_6$ to $C_{30}$ alkyl, $R_1$ and $R_2$ independently are selected from the group consisting of ketoalkyl, alkoxy, nitro, halogen, and acyloxymethyl (including alkanoyloxymethyl).

In a preferred embodiment, the nitration is conducted with fuming nitric acid as a nitrating agent.

In accordance with the invention, the said alkyl group R contains 6 to 30 carbon atoms.

Thus, the invention described herein makes possible the objectives of:

(1) providing a method for the preparation of a photochromic material possessing absorption sensitivity in the long wavelength region; and

(2) providing a convenient method for the preparation of a photochromic material from which thin films can be formed by the LB method.

This invention may be better understood and its numerous objects and advantages will become apparent to those skilled in the art by reference to the accompanying drawing as follows:

Figure 1 shows the ultraviolet and visible absorption spectra of the colorless form and colored form of the spiropyran compound NSP1822 prepared by the method of the present invention in an LB film.

The invention is further explained in detail by way of the Examples which follow.

Example 1

The method for the preparation of the spiropyran compound NSP1822 shown by the formula III will be described below:

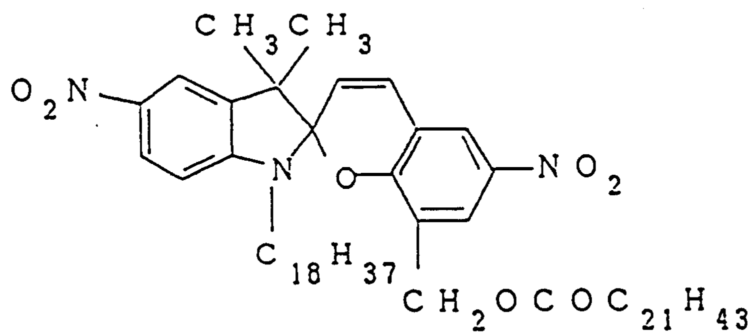

(III)

NSP1822

First, the spiropyran compound shown by the following formula was prepared:

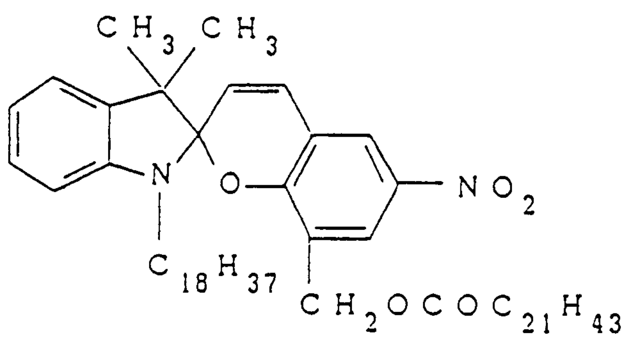

(Step 1)

First, 42.3 g (266 mmol) of 2,3,3-trimethyl indolenine 1 and 101.1 g (266 mmol) of iodooctadecane 2 were dissolved in 200 ml of 2-butanone, then the mixture was heated and refluxed for 40 hours. After distilling off the 2-butanone, the solid residue was recrystallized from 1000 ml of ethanol, thereby obtaining 91.5 g (197 mmol, yield 63.9%) of 1-octadecyl-2,3,3-trimethylindolenium iodide 3 in the form of a reddish-white solid. This reaction can be expressed by the following chemical equation:

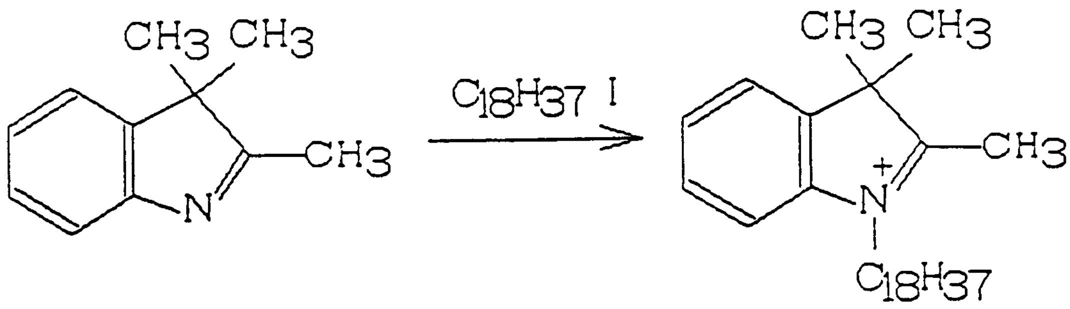


1 2 3

(Step 2)

First, 91.5 g (197 mmol) of 1-octadecyl-2,3,3-trimethylindolenium iodide 3 obtained in Step 1 was dispersed in 100 ml of diethyl ether, and this in turn was dispersed in 400 ml of a 3.8 N aqueous solution of sodium hydroxide. This suspension was then agitated for 3.5 hours, after which the oily layer was extracted with diethyl ether. After being dried over sodium hydroxide for 24 hours, the diethyl ether was distilled off, thereby obtaining 65.6 g (159 mmol, yield 80.7%) of 1-octadecyl-2-methylene-3,3-dimethylindoline 4 in the form of a yellow liquid. This reaction can be expressed by the following chemical equation:

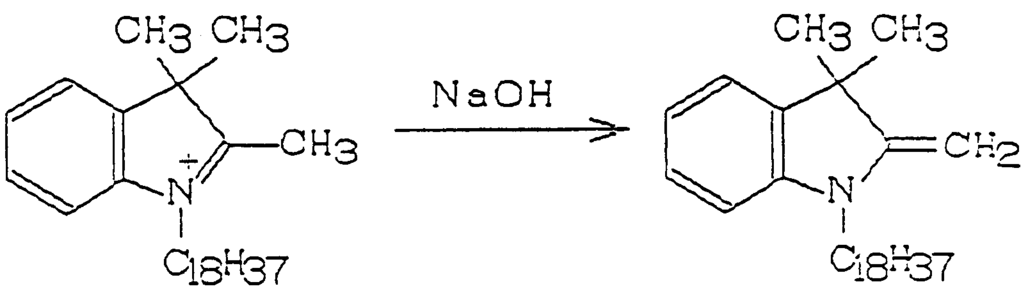


3 4

(Step 3)

First, 11.3 g (52.6 mmol) of 3-chloromethyl-5-methoxysalicylaldehyde 5 was mixed with 20.6 g (52.6 mmol) of silver behenate, benzene was added, then this heterogeneous system was heated and refluxed for 40 hours. The mixture was then filtered and the filtrate was concentrated, after which recrystallization was effected in a mixed benzene-hexane (1:5) solvent, thereby obtaining 11.1 g (21.3 mmol, yield 40.5%) of 3-docosanoyloxymethyl-5-nitrosalicylaldehyde 6 in the form of yellow needle crystal. This reaction can be expressed by the following chemical equation:

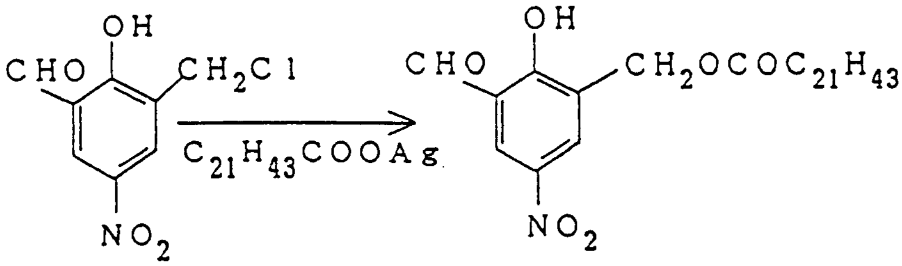


5 6

(Step 4)

First, 2 g (4.9 mmol) of 1-octadecyl-2-methylene-3,3-dimethylindoline 4 obtained in Step 2 above together with 2.1 g (4.1 mmol) of 3-docosanoyloxymethyl-5-nitrosalicylaldehyde 6 obtained in Step 3 above were heated and refluxed in 20 ml of ethanol for 1 hour. Then, the deep green reaction mixture was cooled and the precipitate so obtained was recrystallized three times from 80 ml of ethanol, thereby obtaining 2.5 g (2.7 mmol, yield 65.9%) of the spiropyran compound 7 (obtained as yellowish-brown crystals). This reaction can be expressed by the following chemical equation:

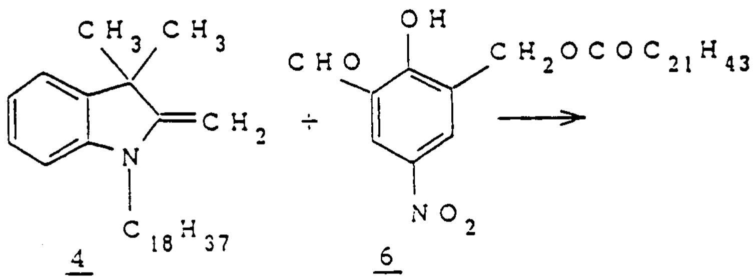

4 6

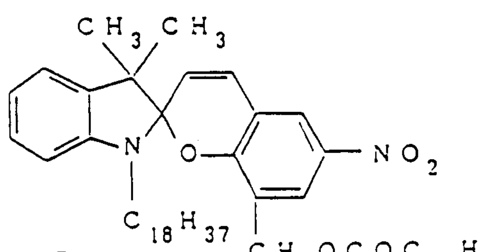

7

The spiropyran compound 7 obtained in this manner was then nitrated by the method of the present invention, as follows.

(Step 5)

Ten milliliters of fuming nitric acid was dissolved in 100 ml of acetic acid, and an acetic acid solution of 2.5 g (2.7 mmol) of the spiropyran compound 7 was added dropwise to this solution. After 15 minutes, this reaction mixture was poured into a mixture of hexane and water. The organic (i.e., hexane) layer was separated, and the water layer was extracted with hexane. The combined organic layer was washed with aqueous sodium hydrogencarbonate, then dried, concentrated and purified by the use of column chromatography. The product was then recrystallized twice from hexane, thereby obtaining 300 mg of the spiropyran compound NSP1822.

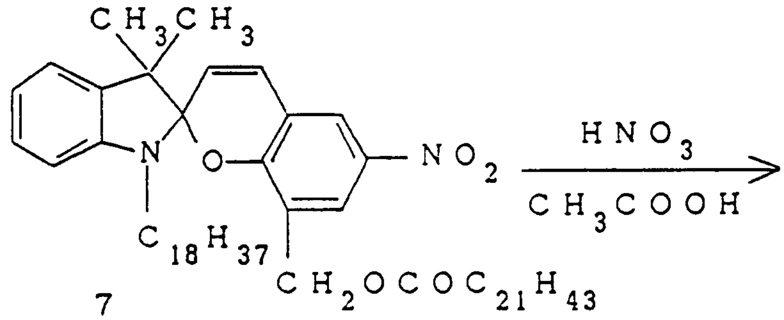


7

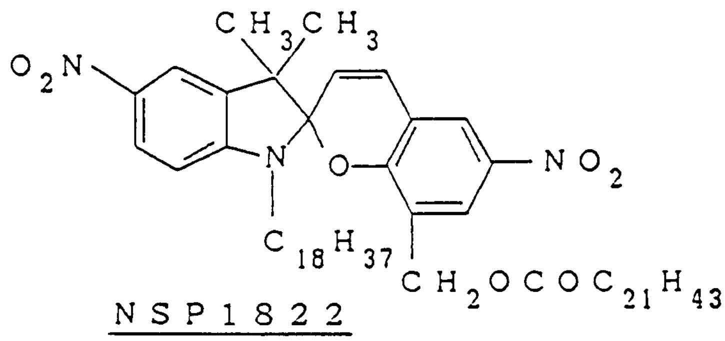

NSP1822

The proton NMR spectrum was measured in order to verify the structure of the reaction product. The result of the measurement is shown in Table 1.

Table 1

| $^1$H-NMR spectrum data of NSP1822 | | | |
|---|---|---|---|
| Chemical shift δ (ppm) | Multiplicity | Assignment | Number of protons |
| 0.88 | t | terminal methyls of long-chain alkyls J = 6.8 Hz | 6 |
| 1.22 | s | 3′-methyl | 3 |
| 1.25 | m | methylenes in long-chain alkyls | 70 |
| 1.34 | s | 3′-methyl | 3 |
| 2.26 | t | methylene binding to ester carbon J = 4.8 Hz | 2 |
| 3.14 | m | methylene binding to the nitrogen of indoline skeleton J = 4.8Hz | 2 |
| 4.91 | s | oxymethylene | 2 |
| 5.86 | d | 3-olefin J = 10.4 Hz | 1 |
| 6.53 | d | 7′-H J = 8.8 Hz | 1 |
| 6.98 | d | 4-olefin J = 10.4 Hz | 1 |
| 7.95 | d | 5-H J = 2.0 Hz | 1 |
| 8.01 | d | 7-H J = 2.4 Hz | 1 |
| 8.11 | d | 4′-H J = 3.2 Hz | 1 |
| 8.18 | d, d | 6′-H J = 8.8, 2.4 Hz | 1 |

In Table 1, the values of chemical shift were measured using TMS as a standard, and multiplicity indicated represents the form of each peak, with "s" denoting singlet, "d" doublet, "t" triplet and "m" multiplet. The parameter J in the Assignment column represents the coupling constant.

Thin films using the compound NSP1822 obtained in this manner were prepared by the LB method under the conditions shown below.

Film composition: NSP1822/octadecane = 1/4

Substrate: quartz substrate with hydrophobic surface obtained by the surface treatment with chlorotrimethylsilane

Number of layers deposited: 4
Compression rate: 10 mm/min
Rate of deposition: 10 mm/min

The films so obtained were extremely thin, with a thickness of approximately 10 nm (100 angstroms), and were of high uniformity. These films were rapidly transformed from the colorless to the colored state by exposure to ultraviolet radiation (366 nm). These colored films have an absorption maximum at the wavelength of 617 nm, the wavelength of which is in a longer wavelength region as compared with storage media employing conventional spiropyran compounds. Moreover, the colored form of the present compound is extremely stable, and displayed no change in absorbance even if the film is allowed to stand in a dark place at room temperature for 24 hours. This colored film was transformed to the colorless form by irradiation with a semiconductor laser beam with a wavelength of 670 nm and output power 10 mW. Thus, the compound NSP1822 obtained by the method of the present invention can be formed into films by the LB method, moreover, the said films are of high stability, permit the recording of information using semiconductor lasers, and thus the compound NSP1822 is preferably used in optical storage media.

A 1:1 (weight ratio) mixture of the aforementioned compound, NSP1822, and polymethylmethacrylate was dissolved in methylene chloride, and a film was formed by spin coating this mixture onto a quartz substrate at 2000 rpm. This film displayed similar photochromism. Films of this material can also be formed by other methods, and such films likewise display similar photochromism.

Spiropyran compounds with a hexyl or triacontyl substituent group at the 1' position were also synthesized by procedures similar to those described above. Moreover, spiropyran compounds with an methoxy group, acetyl group, acetyloxymethyl group or chlorine atom in place of the heneiconsanoyloxymethyl group at the 8 position were also synthesized by similar methods. The wavelengths of the absorption maxima of the colored forms of these various compounds are shown in Table 3.

Example 2

The spiropyran compound NSP1801b shown by the formula IV was synthesized by the method described below.

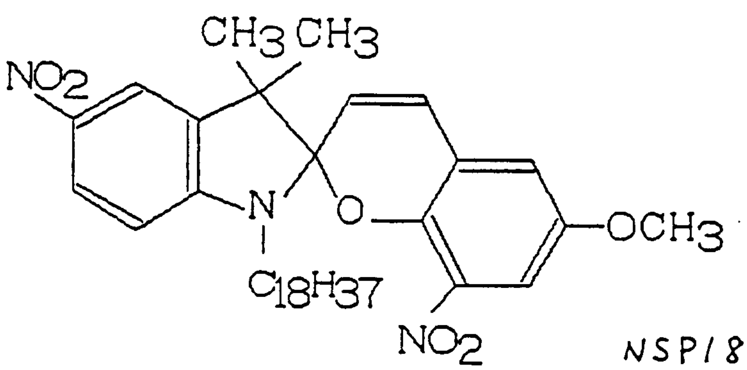

(IV)

NSP1801b

First, the spiropyran compound shown by the formula 10 was synthesized by the following method.

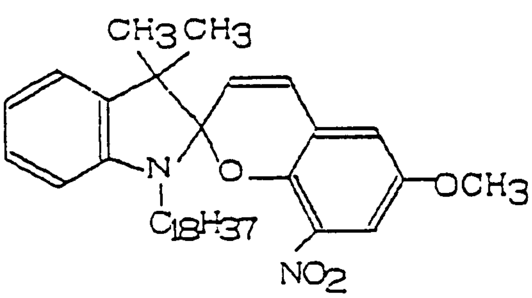

10

(Step 1)

First, 8.0 g (52.6 mmol) of 5-methoxysalicylaldehyde 8 was dissolved in 50 ml of acetic acid. Then, a mixed solution of 2.5 ml (59.7 mmol) of fuming nitric acid (99%, specific gravity 1.52) and 8 ml of acetic acid was added dropwise into the above mixture over a period of 1 hour while maintaining the temperature of the reaction mixture at approximately 15 °C with ice-cold water and strongly agitating the reaction mixture. Agitation was further continued for 7 hours. The precipitate formed was filtered, after which the precipitate was recrystallized from 500 ml of ethanol, thereby obtaining 4.2 g (21.3 mmol, yield 40.5%) of 3-nitro-5-methoxysalicylaldehyde 9 in the form of yellow needle crystals. This reaction can be expressed by the following chemical equation:

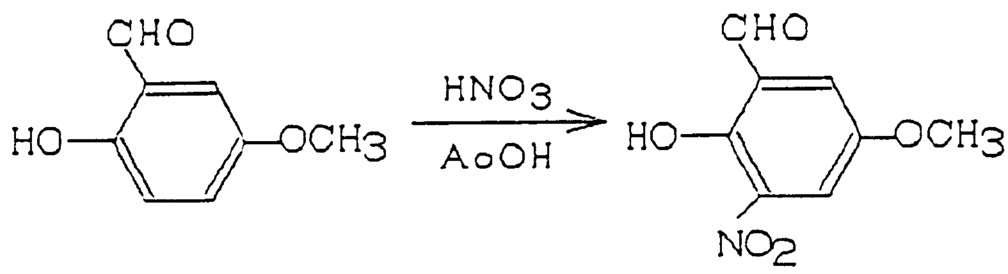


8 9

(Step 2)

First, 2 g (4.9 mmol) of 1-octadecyl-2-methylene-3,3-dimethylindoline 4 synthesized in Step 2 of Example 1 and 0.8 g (4.1 mmol) of 3-nitro-5-methoxysalicylaldehyde 9 synthesized in Step 1 of the present example were heated and refluxed in 20 ml of ethanol for 1 hour. The deep green reaction mixture was then cooled and the precipitate so obtained was recrystallized three times from 80 ml of ethanol, thereby obtaining 1.6 g (2.7 mmol, yield 65.9%) of the spiropyran compound 10 (yellowish-brown crystals). This spiropyran compound 10 possesses a methoxy group at the 6 position, a nitro group at the 8 position and an octadecyl group at the 1' position of the spiropyran skeleton. This reaction can be expressed by the following chemical equation:

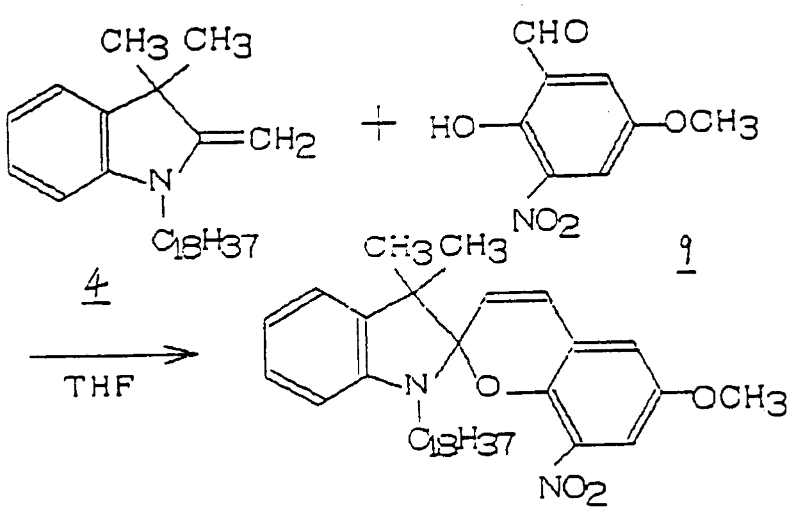


4 9

10

The spiropyran compound 10 obtained in this manner was then nitrated by the method of the present invention, as follows.

(Step 3)

First, 1.6 g (2.7 mmol) of the spiropyran compound 10 obtained in Step 2 above was dissolved in 10 ml of acetic acid. Then a mixed solution of 0.25 ml (6.0 mmol) of fuming nitric acid (99%, specific gravity 1.52) and 1 ml of acetic acid was added dropwise to the above mixture over a period of 30 minutes. One hour later, this reaction mixture was poured into a mixture of hexane and an aqueous solution of sodium hydroxide. The organic (i.e., hexane) layer was separated, and the water layer was extracted with hexane. The combined organic layer was dried, concentrated and then purified by the use of column chromatography. The purified product was then recrystallized twice from ethanol, thereby obtaining 300 mg of the spiropyran compound NSP1801b. This reaction can be expressed by the following chemical equation:

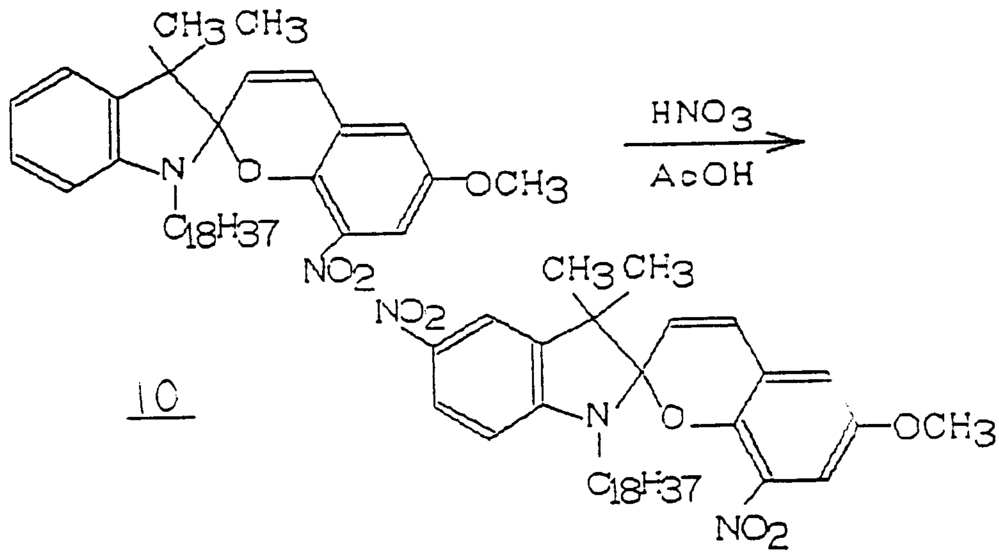


10

NSP1801b

The proton NMR spectrum was measured in order to verify the structure of the reaction product. The result of the measurement is shown in Table 2.

Table 2

| $^1$H-NMR spectrum data of NSP1801b | | | |
|---|---|---|---|
| Chemical shift δ (ppm) | Multiplicity | Assignment | Number of protons |
| 0.88 | t | terminal methyl of long-chain alkyl J = 5.1 Hz | 3 |
| 1.20 | s | 3′-methyl | 3 |
| 1.25 | m | methylenes in long-chain alkyls | 32 |
| 1.38 | s | 3′-methyl | 3 |
| 3.24 | t | methylene binding to the nitrogen of indoline skeleton J = 7.1 Hz | 2 |
| 3.80 | s | methoxy | 3 |
| 5.85 | d | 3-olefin J = 10.3 Hz | 1 |
| 6.49 | d | 7′-H J = 8.6 Hz | 1 |
| 6.68 | d | 4-olefin J = 10.3 Hz | 1 |
| 6.90 | s | 5-H | 1 |
| 7.24 | s | 7-H | 1 |
| 7.92 | s | 4′-H | 1 |
| 8.14 | d | 6′-H J = 8.6 Hz | 1 |

In Table 2, the values of chemical shift were measured using TMS as a standard, and multiplicity indicated represents the form of each peak, with "s" denoting singlet, "d" doublet, "t" triplet and "m" multiplet.

Thin films using this spiropyran compound could be prepared in the same manner as Example 1. The thin films were initially colorless, and were rapidly transformed to the colored state by exposure to ultraviolet radiation (366 nm). The colored film has an absorption maximum at 660 nm. This colored film is transformed to the colorless state by irradiation with a semiconductor laser beam with a wavelength of 700 nm and output power 10 mW.

Spiropyran compounds with a hexyl or triacontyl substituent group at the 1' position of the spiropyran skeleton were synthesized by procedures similar to those described above. The wavelength of the absorption maxima of the colored forms of these various compounds are shown in Table 3.

Table 3

| Absorption maxima of spiropyran compounds | | | |
|---|---|---|---|
| R | $R_1$ | $R_2$ | Absorption maximum (nm) |
| $C_6H_{13}$ | $NO_2$ | $CH_2OCOC_{21}H_{43}$ | 615 $^{1)}$ |
| $C_{18}H_{37}$ | $NO_2$ | $CH_2OCOC_{21}H_{43}$ | 615 $^{1)}$ |
| $C_{30}H_{61}$ | $NO_2$ | $CH_2OCOC_{21}H_{43}$ | 613 $^{1)}$ |
| $C_{18}H_{37}$ | $NO_2$ | $CH_2OCOCH_3$ | 615 $^{1)}$ |
| $C_{18}H_{37}$ | $NO_2$ | $COCH_3$ | 620 $^{1)}$ |
| $C_{18}H_{37}$ | $NO_2$ | $OCH_3$ | 615 $^{1)}$ |
| $C_{18}H_{37}$ | $NO_2$ | Cl | 610 $^{1)}$ |
| $C_6H_{13}$ | $OCH_3$ | $NO_2$ | 660 $^{2)}$ |
| $C_{18}H_{37}$ | $OCH_3$ | $NO_2$ | 660 $^{2)}$ |
| $C_{30}H_{61}$ | $OCH_3$ | $NO_2$ | 662 $^{2)}$ |

1) in THF
2) in ethanol

Comparative Example

Iodooctadecane and 2,3,3-trimethyl-6-nitro-indolenine 11 were dissolved in 2-butanone, and this solution was heated and refluxed for 40 hours, but no reaction whatsoever occurred.

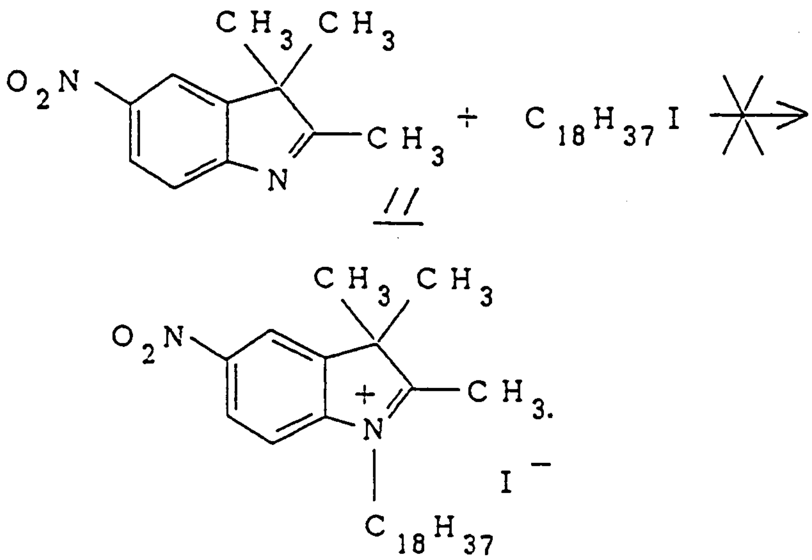

Thus, if nitration of the indoline compound is performed prior to the coupling reaction (e. g., the reactions of Step 4 of Example 1 and Step 2 of Example 2), as in conventional methods, then subsequent reactions for the purpose of introducing an alkyl group at the 1 position of the indoline ring do not proceed, and therefore, the desired type of spiropyran compound cannot be obtained. This is due to the lowering of the activity of the nitrogen atom of the indoline ring resulting from the electron attractive properties of the nitro group. Such a nitrated indoline ring does not react at all with halogenated alkanes having six or more carbon atoms.

By the method of the present invention, spiropyran compounds with a nitro group at the 5' position and a $C_6$ to $C_{30}$ alkyl group at the 1' position can be obtained with high efficiency. In particular, by comparison with previous methods, the method of the present invention is exceedingly effective for synthesis of spiropyran compounds having substituent alkyl groups with six or more carbon atoms at the 1' position.

Fuming nitric acid is used as the nitrating agent in the foregoing examples, however, the use of sulfuric acid-nitric acid mixtures is also similarly effective for the present purpose.

## Claims

**1.** A method for preparing a photochromic material, comprising the step of subjecting a spiropyran compound of formula I to direct nitration to form a nitrated spiropyran compound of formula II which functions as a photochromic material:

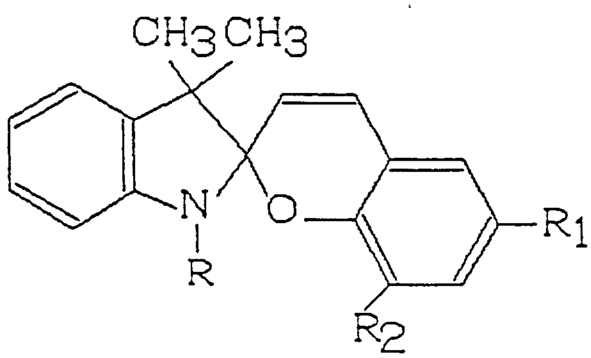

(I)

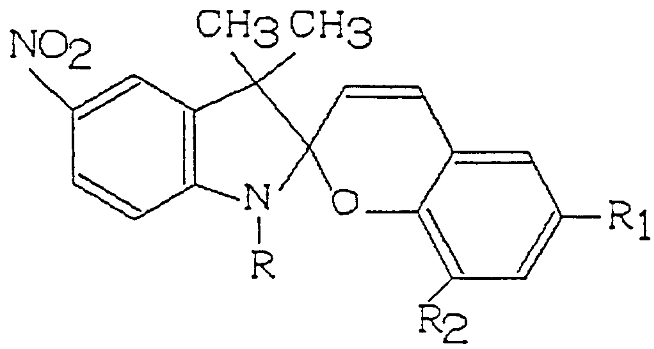

(II)

wherein R is $C_6$ to $C_{30}$ alkyl, $R_1$ and $R_2$ independently are selected from the group consisting of ketoalkyl, alkoxy, nitro, halogen, and acyloxymethyl (including alkanoyloxymethyl).

2. A method according to claim 1, wherein said nitration is conducted with fuming nitric acid as a nitrating agent.

**Patentansprüche**

1. Verfahren zur Herstellung eines photochromen Materials, welches die Maßnahme umfaßt, eine Spiropyranverbindung der Formel I zur Darstellung einer nitrierten Spiropyranverbindung der Formel II, die eine Aktivität als photochromes Material aufweist, einer Direktnitrierung zu unterwerfen:

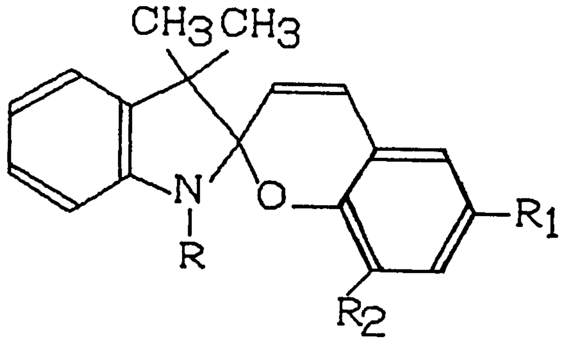

(I)

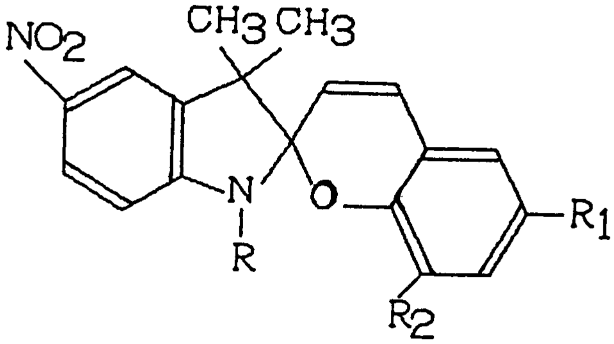

(II)

in der R $C_6$-$C_{30}$-Alkyl bedeutet, wobei $R_1$ und $R_2$ unabhängig voneinander aus der aus Ketoalkyl, Alkoxy, Nitro und Acyloxymethyl (einschließlich des Alkanoyloxymethylrests) bestehenden Gruppe ausgewählt sind.

2. Verfahren nach Anspruch 1, bei dem die Nitrierung mit rauchender Salpetersäure als Nitrierungsmittel durchgeführt wird.

**Revendications**

1. Procédé de préparation d'un matériau photochromique, comportant l'étape consistant à soumettre un composé du spiropyranne de la formule I à une nitration directe pour former un composé nitré du spiropyranne de la formule II qui fonctionne comme matériau photochromique:

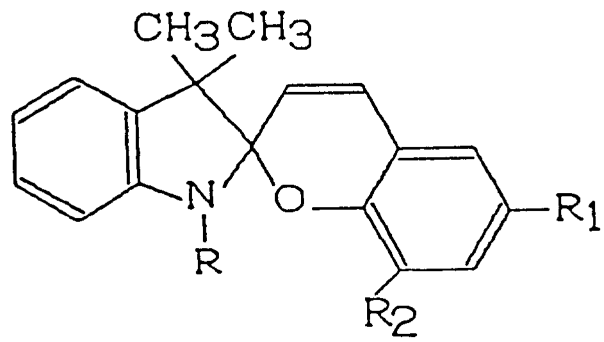

(I)

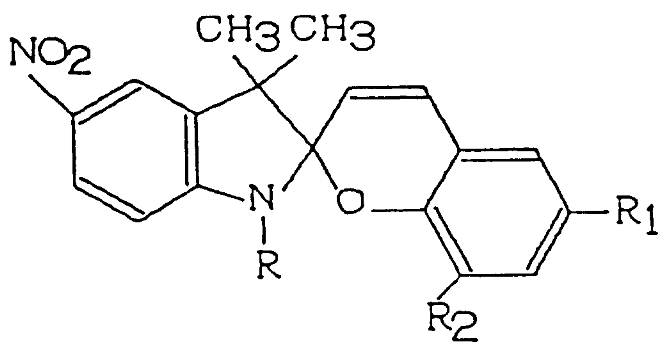

(II)

où R est un alkyle $C_6$ à $C_{30}$, $R_1$ et $R_2$ sont, indépendamment, choisis dans l'ensemble constitué des groupes cétoalkyle, alkoxy, nitro, halogène et acyloxyméthyle (y compris alcanoyloxyméthyle).

2. Procédé selon la revendication 1, dans lequel ladite nitration s'effectue avec de l'acide nitrique fumant comme agent nitrant.

Fig. 1

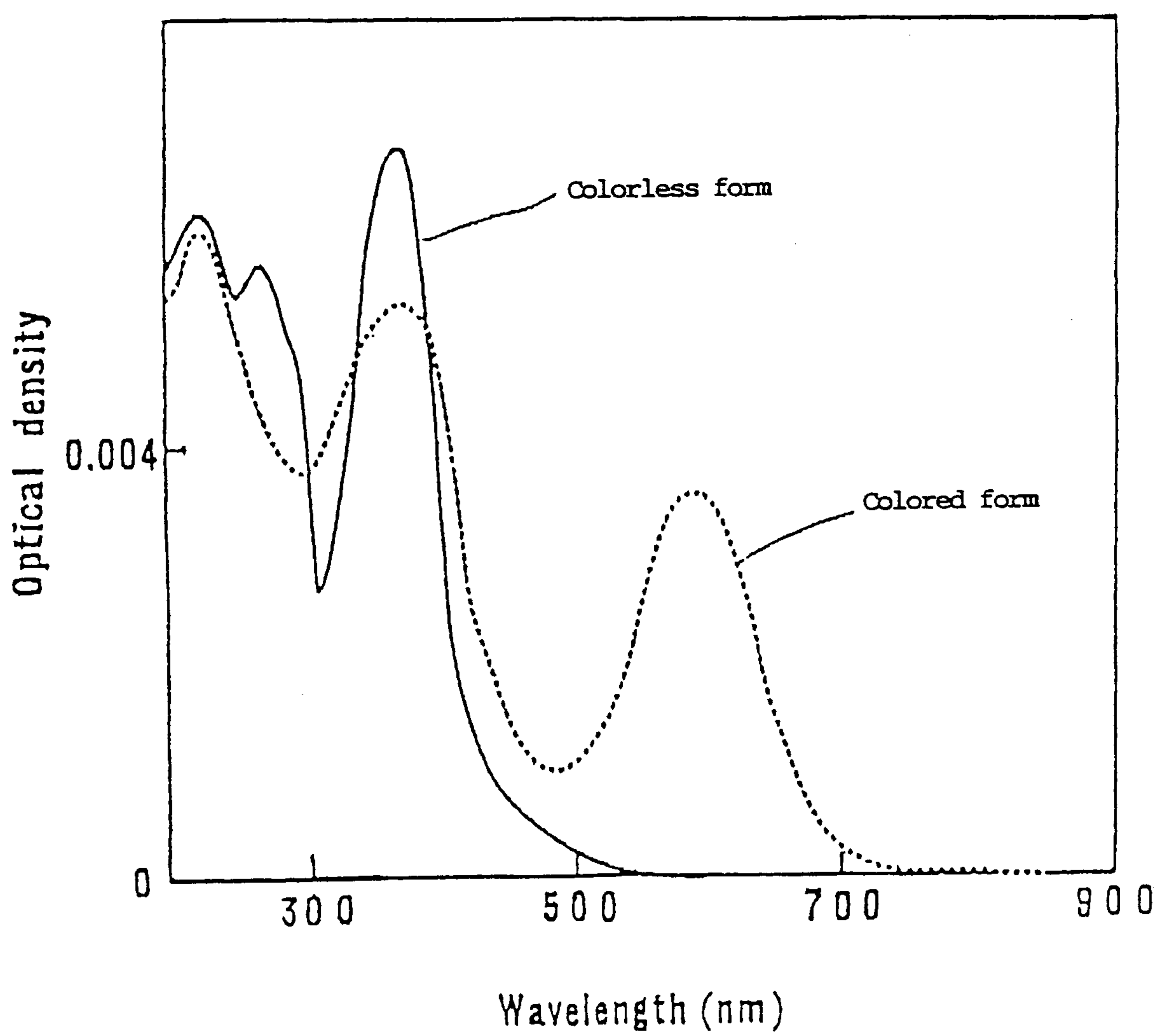
Colorless form
Colored form
Optical density
0.004
0
300
500
700
900
Wavelength (nm)